# EUROPEAN PATENT SPECIFICATION

(11) **EP 4 626 904 B1**
(45) Date of publication and mention of the grant of the patent: **09.09.2026**
(21) Application number: 23814459.6
(22) Date of filing: 30.11.2023
(51) Int. Cl.: C07K 5/09, A61K 8/64, A61K 8/365, A61Q 19/00

(54) **MANDELIC ACID SALT OF PALMITOYL LYSYLVALYL-LYSINE**
MANDELSÄURESALZ VON PALMITOYL LYSYLVALYL-LYSIN
SEL D'ACIDE MANDÉLIQUE DE PALMITOYL LYSOLYSYL LYSINE

(30) Priority: 02.12.2022 EP 22211211
(43) Date of publication of application: 08.10.2025
(73) Proprietor: DSM IP Assets B.V., 6221 BE Maastricht (NL)
(72) Inventor: DOPPLER, Stephan, 4303 Kaiseraugst (CH); HEIDL, Marc, 4303 Kaiseraugst (CH); KOHLER, Lise Anne, 4303 Kaiseraugst (CH)
(74) Representative: dsm-firmenich IP
(86) International application number: PCT/EP2023/083640
(87) International publication number: WO 2024/115619

(56) References cited:
- US-A1- 2007 099 842

## Description

The present invention relates to the mandelic acid addition salt of palmitoyl lysylvalyl-lysine as well as to cosmetic preparation comprising said salt. Furthermore, the invention relates to a to the use of mandelic acid to prepare the mandelic acid addition salt of palmitoyl lysylvalyl-lysine to increase the storage stability of palmitoyl lysylvalyl-lysine.

The prominent role of peptides in nature makes them valuable cosmetic ingredients. Cosmetic peptides are involved in many physiological processes (e.g. increase of collagen production) and have various benefits (e.g. anti-wrinkle, pigmentation reduction, firming).

Palmitoyl-lysylvalyl-lysine bistrifluoroacetate, commercially available as SYN^{®}-COLL from DSM Nutritional Products Ltd, is the bistrifluoroacetic acid addition salt of the synthetic tripeptide Pal-KVK and is widely used in cosmetic applications.

Trifluoroacetic acid (TFA) is typically used in the peptide synthesis during the process of cleaving peptides from their respective resin, as well as an ion pairing agent in HPLC purifications. Therefore synthesized peptides are usually in the form of TFA salts (up to 45% TFA content). In addition the TFA salts generally exhibit a good solubility, which is a prerequisite for cosmetic applications.

However, cosmetic compositions comprising TFA respectively salts thereof are not well appreciated anymore by all consumers.

Thus, there is an ongoing need for alternative peptide salts, which are free of TFA, but still exhibit a sufficient solubility while also providing a good storage stability.

It has now surprisingly been found that the mandelic acid addition salt of palmitoyl lysylvalyl-lysine overcomes the drawbacks of the prior art.

Thus, in a first embodiment the invention relates to the mandelic acid addition salt of palmitoyl lysylvalyl-lysine (Pal-KVK).

The term 'palmitoyl lysylvalyl-lysine' includes all possible isomeric forms as well as mixtures thereof, e.g. racemic mixtures and mixtures of rotamers.

In all embodiments of the present invention, palmitoyl lysylvalyl-lysine is preferably palmitoyl L-lysyl-L-valyl-L-Lysine (Cas No: 623172-55-4; also known as N²-(1-oxohexadecyl)-L-lysyl-L-valyl-L-Lysine).

It is well understood, in all embodiments of the present invention that mandelic acid can be used as D-(-)-mandelic acid (CAS No 611-71-2), L-(+)-Mandelic acid (CAS No. 17199-29-0) or D, L-mandelic acid (CAS No 611-72-3), preferably D, L-mandelic acid is used in all embodiments of the present invention.

Preferably, the mol equivalents of mandelic acid to palmitoyl lysylvalyl-lysine is selected in the range of 0.5 to 3 mol equivalents, preferably in the range of 0.75 to 2.5 mole equivalents, most preferably in the range of 0.8 to 2.25 mole equivalents, such as in the range of 1.75 to 2.25 mole equivalents, based on the palmitoyl lysylvalyl-lysine. Further suitable ranges are from 0.75 to 1.5 mole equivalents or from 1 to 1.25 mole equivalents.

Preferably, in all embodiments of the present invention, the mandelic acid addition salt of palmitoyl lysylvalyl-lysine is N²-(1-oxohexadecyl)-lysyl-valyl- lysine mandelic acid (mol equivalents peptide/ acid about 1:2), most preferably N²-(1-oxohexadecyl)-L-lysyl-L-valyl- L-Lysine D, L-mandelic acid (mol equivalents peptide/ acid about 1:2).

In all embodiments of the present invention, the mandelic acid salt of palmitoyl lysylvalyl-lysine is preferably prepared by dissolving the respective acetate salt of palmitoyl lysylvalyl-lysine (e.g. prepared as outlined in example 6 of US2007/0099842) in a suitable solvent forming a peptide acetate solution, combining said peptide acetate solution with a solution of mandelic acid dissolved in a solvent (either by addition of said peptide solution to the mandelic acid solution or the other way round), followed by removal of said solvent(s) by suitable means such as evaporation and/ or lyophilization.

Suitable solvents include in particular alkyl alcohols such as C₁₋₆ alkyl alcohols e.g. including methanol, ethanol, propanol, isopropanol and acetic acid, as well as mixtures thereof with methanol and acetic acid as well as any mixtures thereof being particularly preferred.

The amount of solvent is preferably selected such, that the palmitoyl lysylvalyl-lysine acetate and the mandelic acid are fully dissolved.

Thus, in a particular preferred embodiment, the mandelic acid salt of palmitoyl lysylvalyl-lysine with all the definitions and preferences as given herein is prepared by
(i) Dissolving the respective palmitoyl lysylvalyl-lysine acetate in a solvent with all the definitions and preferences as given herein to form a peptide acetate solution,
(ii) Combining the peptide acetate solution with a solution of the respective mandelic acid dissolved in a solvent with all the definitions and preferences as given herein,
(iii) Removing said solvent(s) by evaporation, optionally and preferably followed by lyophilization.

In a preferred embodiment, the mandelic acid addition salt of palmitoyl lysylvalyl-lysine with all the definitions and preferences as given herein is comprised in a composition further comprising at least one water-soluble polyol containing 2 to 10 carbon atoms and 2 to 7 hydroxyl groups and optionally water (also referred to herein as peptide composition).

In a particular advantageous embodiment the peptide composition according to the present invention comprises
(i) 0.001-12.5 wt.-%, particularly 0.01-2 wt.-%, most particularly 0.1-0.5 wt.-% of the mandelic acid addition salt of palmitoyl lysylvalyl-lysine with all the definitions and preferences as given herein,
(ii) up to 99.999 wt.-%, particularly 40-80 wt.-%, most particularly 60-75 wt.-% of at least one water-soluble polyol containing 2 to 10 carbon atoms and 2 to 7 hydroxyl groups and optionally
(iii) up to 90 wt.-%, particularly up to 50 wt.-%, most particularly 0-35 wt.-% of water.

Even more preferably, the peptide composition according to the present invention comprises
(i) from about 1000 to about 2000 ppm of the mandelic acid addition salt of palmitoyl lysylvalyl-lysine with all the definitions and preferences as given herein,
(ii) from about 60 to about 75 wt.-% of glycerin and/ or 1,3-propanediol, and
(iii) from about 24.9 to about 39.9 wt.-% of water.

In a particular advantageous embodiment, the ingredients (i) to (iii) in the peptide composition sum up to 100 wt.-%.

The peptide compositions according to the invention may further contain tensides and/ or thickeners. Suitable tensides to be used in said peptide compositions are the ones appropriate for cosmetic applications. Said tensides are well known to a person skilled in the art and include in particular non-ionic tensides such as e.g. Polysorbate-20. Suitable thickeners to be used in said peptide compositions are again the ones appropriate for cosmetic applications such as e e.g. polyacrylic acids (Carbomers).

In another embodiment the invention relates to the use of mandelic acid to increase the storage stability of palmitoyl lysylvalyl-lysine with all the definitions and preferences as given herein, preferably in a peptide composition comprising at least one water-soluble polyol containing 2 to 10 carbon atoms and 2 to 7 hydroxyl groups with all the definitions and preferences given herein and optionally water and/ or a topical preparation as defined herein.

The increase in storage stability as defined herein refers to an increased stability after 3 months storage at 4°C, 22 °C (RT) and 40°C, compared e.g. to the respective methansulfonylsulfonic acid salt (preferably in glycerin) or 2-methoxy-2-phenylacetic acid addition salt (preferably in propanediol).

Preferably, the increase in the storage stability @ RT after 3 months compared e.g. to the respective methansulfonylsulfonic acid salt in glycerin or 2-methoxy-2-phenylacetic in propanediol is at least 10%, more preferably at least 15 %, most preferably at least 20%.

The water-soluble polyol containing 2 to 10 carbon atoms and 2 to 7 hydroxyl groups is in particular selected from ethylene glycol, 1,2-propylene glycol, 1,3-propanediol (also referred herein to as propanediol), 1,4-butylene glycol, glycerin, erythrit (*meso*-1,2,3,4-Butantetrol), sorbit, mannit, methylglucoside, diglycerin, triglycerin and/ or pentaerythrit. Particularly, the polyol is glycerin or 1,3-propanediol, most preferably glycerin.

In a particular embodiment, the addition salt is used in the form of a blend consisting essentially of
(a) 0.05-1 wt.-%, preferably 0.1 to 1 wt.-%, most preferably 0.2 to 0.5 wt.-% of the addition salt according to the present invention,
(b) 15-50 wt.-%, preferably 20 to 40 wt.-%, most preferably 25 to 35 wt.-% of panthenol,
(c) 0.1-1 wt.-%, preferably 0.2 to 0.8 wt.-%, most preferably 0.25 to 0.7wt.-% of sodium hyaluronate,
(d) 0.25-7 wt.-%, preferably 0.3 to 5 wt.-%, most preferably 0.5 to 3 wt.-% of at least one algae extract,
(e) 0.25-7 wt.-%, preferably 0.3 to 5 wt.-%, most preferably 0.5 to 3 wt.-% of pentylene glycol,
(f) up to 1 wt.-%, preferably 0.001 to 1 wt.-% of citric acid, and
(g) >50% water.

It is well understood by a person skilled in the art, that water is used to adjust the blend to 100 wt.-%

The term 'the blend consists essentially of' as used according to the present invention means that the total amount of the listed ingredients ideally sum up to 100 wt.-%. It is however not excluded that small amounts of impurities or additives may be present, with the proviso that the total amount of such impurities or additives is preferably less than 3 wt.-%, more preferably less than 2 wt.-%, most preferably less than 1 wt.-% and which are introduced via the respective raw materials.

It is furthermore well understood, that the (total) amount of water in the blend is the sum of water added via the respective raw materials such as e.g. via an aqueous algae extract and 'free' water.

The blend is preferably a colourless to yellowish liquid and exhibits a pH value of approximately 4.0-6.0, preferably 4.5-5.5 and a viscosity in the range of less than 3000 mPas, preferably less than 2000 mPAS, most preferably less than 1500 mPas (measured with Brookfield RVDV-II+, spindle 3 at 25°C with a shear rate of 10 rpm for 30s).

The algae extract in the blend according to the present invention is preferably an extract of the biotechnologically produced microalgae *Dunaliella Salina*

Such extracts are obtainable by cultivation of the respective algae, followed by harvesting the cells. Afterwards the harvested cells are rehydrated and extracted with hot water. The crude extract is subsequently centrifuged and ultrafiltrated. In the preparation of the blend, the algae extract can be used in dried form or in the form of a concentrated aqueous algae extract with a known solid content. Preferably, the algae extract is added to the blend in the form of a concentrated aqueous algae extract, having a known algae extract content (solid content). Preferably an aqueous algae extract having an algae extract content selected in the range of 0.5 to 10 wt.-%, preferably in the range of 1 to 5 wt.-%, based on the total aqueous algae extract, is used in the preparation of the blends according to the present invention. Suitable algae extract to be used for the preparation of the blend according to the present invention is PEPHA^{®}-CTIVE CB consisting of 1-5% of Dunaliella Salina Extract, 1-5% of Pentylene Glycol, 0.01-0.1 % of citric acid and ad 100% of water.

Panthenol [CAS 81-13-0] is e.g. commercially available as D-panthenol or D-panthenol 75L at DSM Nutritional Products Ltd.

The term sodium hyaluronate [CAS 9067-32-7] as used herein refers to the sodium salt of hyaluronic acid. Preferably low molecular weight sodium hyaluronate is used in the blends according to the present invention such as sodium hyaluronate having a molecular weight (MW) in the range of 10 to 800 kDa, preferably having a MW in the range of 200 to 600kDa and most preferably having a MW in the range of 200 to 400 kDa. Such sodium hyaluronate is e.g. available under the tradename HYA-ACT^{™} S at DSM Nutritional products Ltd as well as BasHyal (MW 100-300kDa) or Renovhyal (MW 15-50 kDa) at Soliance. Most preferred in all embodiments is the use of HYA-ACT^{™} S, a small molecular weight hyaluronic acid of 200-400kDa.

The addition salt, respectively the peptide composition as well as the blend as defined herein can be incorporated into cosmetic preparations useful for improving skin appearance and physiology such as e.g. reducing fine lines, wrinkles and other symptoms associated with aged or photodamaged skin, treatment of stretch marks or tightening, firming and/ or moisturizing skin.

Thus, the invention also relates to a cosmetic preparation comprising an addition salt respectively a peptide composition as defined herein and a cosmetically acceptable carrier.

The term cosmetic preparation as defined herein refers in particular to cosmetic preparations that can be topically applied to mammalian keratinous tissue such as e.g. human skin or hair (including eyelashes, the eyebrows) or the nails, particularly human skin.

The term "cosmetic preparation" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4th edition, 1992.

The term cosmetically acceptable carrier refers to all carriers and/or excipients and/ or diluents conventionally used in cosmetic preparations.

In an advantageous embodiment, the cosmetic preparations according to the present invention comprise from 50% to 99%, preferably from 60% to 98%, more preferably from 70% to 98%, such as in particular from 80% to 95% of a carrier, based on the total weight of the composition.

In a particular advantageous embodiment, the carrier in all embodiments of the present invention consists furthermore of at least 30 wt. %, preferably of at least 40 wt.-%, more preferably of at least 45 wt.-%, most preferably of at least 50 wt.-% of water, such as in particular of 50 to 95 wt.-%, from 50 to 80 wt.-%, from 50 to 70 wt.-% or from 50 to 60 wt.-% of water.

Preferably, the cosmetic preparations are in the form of a suspension or dispersion in solvents or fatty substances, or alternatively in the form of an emulsion (including micro, PIT, pickering and multiple emulsion), such as in particular of O/W- or W/O-type, gels such as hydrogels, alcoholic gels, lipogels, one- or multiphase solution or vesicular dispersion or other usual forms, which can also be applied by pens, as masks or as sprays. If the cosmetic preparation is or comprises an emulsion it can also contain one or more anionic, nonionic, cationic or amphoteric surfactant(s).

Preferred cosmetic preparations are skin care compositions, and functional compositions.

Cosmetic preparation in accordance with the invention can be in the form of a liquid, lotion, a thickened lotion, a gel, a cream, a milk, an ointment, a paste, a powder, a make-up, or a solid tube stick and can be optionally be packaged as an aerosol and can be provided in the form of a mousse such as a aerosol mousse, a foam or a spray foam, a spray, a stick, a plaster, a cleanser, a soap, a wipe or a lyophilizate.

The cosmetic preparation according to the invention are preferably formulated as an oil-in-water or water-in-oil emulsion, water-in-silicone or silicone-in-water emulsion or as an aqueous serum or aqueous gel in particular as an oil-in water emulsion (O/W emulsion).

In another preferred embodiment, the cosmetic preparations according to the present invention are in the form of an aqueous gel and/ or a clear gel.

The cosmetic preparation according to the invention have a pH in the range of 3-10, preferably in the range of pH of 4-8, most preferred in the range of pH 4-6.

In accordance with the present invention, the topical preparation may optionally comprise further ingredients such as ingredients for skin lightening; tanning prevention; treatment of hyperpigmentation; preventing or reducing acne, wrinkles, lines, atrophy and/or inflammation; as well as topical anesthetics; antimicrobial and/or antifungal agents; chelators and/or sequestrants; anti-cellulites and slimming (e.g. phytanic acid), firming, moisturizing and energizing, self-tanning, soothing, as well as agents to improve elasticity and skin barrier and/or UV-filter substances. The topical cosmetic preparations can also contain usual cosmetic adjuvants and additives, such as preservatives/ antioxidants, fatty substances/ oils, water, organic solvents, silicones, thickeners, softeners, emulsifiers, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, e.g. those suited for providing a photoprotective effect by physically blocking out ultraviolet radiation, or any other ingredients usually formulated into cosmetic preparations. Such cosmetic ingredients commonly used in the skin care industry, which are suitable for use in the cosmetic preparation of the present invention are e.g. described in the CTFA Cosmetic Ingredient Handbook, Second Edition (1992) without being limited thereto.

The usual cosmetic adjuvants and additives such as e.g. emulsifiers, thickeners, surface active ingredients and film formers can show synergistic effects which can be determined by the expert in the field with normal trials, or with the usual considerations regarding the formulation of cosmetic preparation.

The necessary amounts can, based on the desired product, easily be determined by the skilled person. The cosmetically active ingredients useful herein can in some instances provide more than one benefit or operate via more than one mode of action.

If nothing else is stated, the carrier, excipients, additives, diluents, adjuvant and additives etc. mentioned in the following are in particular suitable for cosmetic preparation according to the present invention.

The following examples are provided to further illustrate the invention. These examples are illustrative only and are not intended to limit the scope of the invention in any way.

### Examples

| | |
|---|---|
| Abbreviation: | MA = Mandelic acid (Hydroxyphenylessigsäure) |
| | MSA = Methanesulfonic acid |
| | MPAA = 2Methoxy-2-phenylacetic acid |
| | DAB: 1,4-Diaminobutyric acid (building block in the tripeptide) |

### Example 1: Preparation of various Palm-Lys-Val-Lys-OH addition salts

### 1.1 Palm-Lys-Val-Lys-OH *2MA

To a solution of 2g (2.73 mmol) Palm-Lys-Val-Lys-OH *2AcOH in 90% aqueous MeOH 0.852g (5.60 mmol, 2.05 eq) mandelic acid dissolved in 20 ml MeOH was added. The mixture was stirred until a homogenous solution was obtained. Then MeOH was removed first at reduced pressure, followed by lyophilization.

### 1.2 Palm-Lys-Val-Lys-OH *2MPAA (Reference)

To a solution of 500 mg (0.68 mmol) Palm-Lys-Val-Lys-OH *2AcOH in 90% aqueous MeOH 233 mg (1.40 mmol, 2.05 eq) methoxyphenylacetic acid was added. The mixture was stirred until a homogenous solution was obtained. Afterwards the MeOH was removed first at reduced pressure, followed by lyophilization.

### 1.3 Palm-Lys-Val-Lys-OH *2MSA (Reference)

To a solution of 200 mg (0.273 mmol) Palm-Lys-Val-Lys-OH *2AcOH in 90% aqueous MeOH 53.8 mg (0.56 mmol, 2.05 eq) methanesulfonic acid (MSA) was added. The mixture was stirred until a homogenous solution was obtained. Afterwards MeOH was removed first at reduced pressure, followed by lyophilization.

### Example 2: Preparation of the peptide compositions

### 2.1 Palm-Lys-Val-Lys-OH *2MA - glycerin/water composition (MA-Gly)

100mg Palm-Lys-Val-Lys-OH *2MA prepared as outlined above was charged into a 100ml round bottom necked flask. 21.0g (from total 24.9g) water is added and the peptide is dissolved at 85°C using short time (approx. 5-15 sec) ultrasonic bath. Afterwards 58.1g Glycerin (99.9%) is added (pH adjusted to approx. 4.5 with aqueous NaOH (aprox. 0.05M)), followed by the addition of 3.36g of water.

### 2.2 Palm-Lys-Val-Lys-OH *2MA - 1,3-propanediol (PDO)/water composition (MA-PDO)

100mg Palm-Lys-Val-Lys-OH *2mandelate was charged into a 100ml round bottom necked flask. 21.4g (from total 24.9g) water is added and the peptide is dissolved at 85°C using short time (approx. 5-15 sec) ultrasonic bath. 58.1g PDO (99.9%) is added (pH approx. 4.5) followed by the addition of 3.5g of water.

### 2.3 Palm-Lys-Val-Lys-OH *2MSA - glycerin/water composition (MSA-Gly)

200mg Palm-Lys-Val-Lys-OH *2MSA is charged into a 250ml round bottom necked flask. 33.2g water is added and the peptide is dissolved at approx..80°C. Then 132.3g Glycerin (86.5%) is added (pH is adjusted to approx. 4.5 using aqueous NaOH (approx. 0.05M).

### 2.4 Palm-Lys-Val-Lys-OH *2MPAA - 1,3-propanediol (PDO)/water composition (MPAA-PDO)

100mg Palm-Lys-Val-Lys-OH *2MPPA is charged into a 100ml round bottom necked flask. 21.7g water is added and the peptide is dissolved at 85°C using short time (approx. 5-15 sec) ultrasonic bath. 58.1g PDO (99.9%) is added (pH approx. 4.5) followed by addition 3.2g of water.

### Example 3: Storage stability

The colourless solutions of the various salts of the tripeptide palmitoyl lysylvalyl-lysine in glycerin respectively 1,3-propanediol prepared as outlined above (targeted initial peptide content: approx. 1200ppm) were stored at 4 °C, 22°C (RT) and 40°C. After 3 months storage, samples were analyzed for their peptide content with analytical HPLC. In addition the color was assessed, i.e. if it remained colorless or turned yellow(sih). The results are depicted in table 1.

**Table 1-a: peptide content after storage at different temperatures**

| Composition | MA-Gly | MA-PDO | MSA-Gly | MPAA-PDO |
|---|---|---|---|---|
| Salt | MA | MA | MSA | MPAA |
| Solvent | glycerin | propanediol | glycerin | propanediol |
| 4°C | 1018 | 1027 | 939 | 778 |
| RT | 1047 | 1001 | 816 | 320 |
| 40°C | 992 | 1003 | 527 | 53 |
| Color | colorless | colorless | yellow | yellow |

As can be retrieved from table 1-a, the MA salt according to the present invention exhibits a good stability upon storage while the respective MSA or MPAA salts exhibit a significant loss of the active upon storage.

As comparative data, the mandelic acid salt of tetradecyl Dab-Val-Dab-OH (also known as tetradecyl aminobutyroylvalylaminobutyric urea mandelate) and the respective peptide compositions in glycerin and propanediol were prepared as outlined above (referred to as TDVD-MA-Gly & TDVD-MA-PDO). Samples were analyzed for their peptide content after 2 weeks storage at 40°C with analytical HPLC. The results are depicted in table 1-b.

**Table 1-b: peptide content after storage at 40°C for 2 weeks**

| Tripeptide | MA-gly | MA-PDO | TDVD-MA-Gly | TDVD-MA-PDO |
|---|---|---|---|---|
| Salt | MA | MA | MA | MA |
| Solvent | glycerin | propanediol | glycerin | propanediol |
| 40°C/ 2 week | 1033 | 1036 | 660 | 225 |

As can be retrieved from table 1-a and 1-b, the MA salt according to the present invention exhibits a good storage stability, while tetradecyl Dab-Val-Dab-OH mandelate exhibits a significant loss of active already after 2 weeks.

### Example 4 : Stability in a cosmetic preparation

**Table 2: Formulation (clear gel)**

| **Phase** | **INCI Name** | **%w/w** |
|---|---|---|
| A | AQUA | ad 100 |
| | GLYCERIN; AQUA | 4.00 |
| | PHENOXYETHANOL; ETHYLHEXYLGLYCERIN | 1.00 |
| | ACRYLATES/C10-30 ALKYL ACRYLATE CROSSPOLYMER | 0.45 |
| B | **Peptide composition** | 2.00 |
| C | SODIUM HYDROXIDE; AQUA | As needed to pH 6.0 |

**Table 3: peptide content after storage at different temperatures after 3 months (Theoretical initial content in gel: 21.0 ppm for MA-Gly and 20.7 ppm for MA-PDO)**

| Composition | MA-Gly | MA-PDO |
|---|---|---|
| Salt | MA | MA |
| Solvent | glycerin | propanediol |
| 4°C | 20.1 | 19 |
| RT | 19 | 18.1 |
| 40°C | 16.3 | 12.7 |

As can be retrieved from Table 3, the use of the glycerin composition leads to an improved stability in the final formulation compared to the use of the 1,3-propanediol composition.

**Table 4: peptide content after storage at different temperatures after 4 weeks (Theoretical initial content: 19.3 ppm)**

| Composition | MPAA-Gly |
|---|---|
| Salt | MPAA |
| Solvent | glycerin |
| 4°C | 20.8 |
| 40°C | 9.8 |

As can be retrieved from table 3 and 4, the MA salt according to the present invention exhibits a significantly better stability upon storage compared to the respective MPAA salt, which already after 1 months exhibits a loss of active of 52% (at 40°C) whereas the corresponding MA salt only exhibits a loss of -19% after 3 months storage at 40°C.

### Example 5: Blend

A blend consisting essentially of 1.25g of the mandelic acid salt of palmitoyl lysylvalyl-lysine (D/L form), 150 g of D-panthenol (DSM Nutritional Products Ltd), 2.5g Hyaluronic acid (HYA-ACT S, DSM Nutritional Products Ltd), 288 g PEPHA-CTIVE CB (from DSM Nutritional Products Ltd), 25 g of Pentylene Glycol (Hydrolite 5 green) and 55g Milli Q water was prepared. The blend showed an excellent storage stability and recovery of the peptide after storage (2256 ppm after storage at 40°C for 3 months).

### Example 5: Exemplary cosmetic preparations

Table 5 outlines exemplary O/W emulsions, comprising peptide compositions according to the present invention.

**Table 5: Formulation (O/W emulsions)**

| **O/W Emulsions** | **1** | **2** | **3** | **4** | **5** | **6** | **7** | **8** |
|---|---|---|---|---|---|---|---|---|
| Glyceryl Stearate | 2.5 | 2 | 1.2 | 1 | | | 1 | 1 |
| PEG-40 Stearate | 1 | | | | | | | |
| PEG-100 Stearate | | 2.5 | | | | | | 1 |
| Ceteareth-20 | | | | | 1 | | | |
| Glyceryl Stearate Citrate | | | | | | 0.5 | | |
| Potassium Cetyl Phosphate | | | | | | | 3 | 1.5 |
| Stearic Acid | | | 2.5 | 3 | | | | |
| Cetearyl Alcohol | 4 | | | 2 | | | 2 | |
| Stearyl Alcohol | | 2 | 1 | | | | | |
| Cetyl Alcohol | | | 1 | 1 | | | | 0.5 |
| Acrylates/C₁₀₋₃₀ Alkyl Acrylate Crosspolymer | | | | 0.2 | 0.2 | 0.4 | 0.2 | |
| Carbomer | 0.1 | | 0.2 | | | | | |
| Xanthan Gum | | 0.3 | | | | | | 0.3 |
| C₁₂₋₁₅ Alkyl Benzoate | 5 | | | 2 | 5 | 5 | 10 | 5 |
| Petrolatum | 5 | | 3 | | | | | |
| Butylene Glycol Dicaprylate/Dicaprate | | 4 | 2 | | 9 | | | 9 |
| Hydrogenated Polydecene | | | 3 | | 2 | | | 2 |
| Caprylic/Capric Triglyceride | 1 | 3 | | 5 | | 5 | 5 | |
| Cyclomethicone | | 5 | 2 | | | 10 | | |
| Methylpropanediol | 2 | | | | 3 | | | 3 |
| Glycerin | 4 | 7 | 3 | 4 | 3 | | 5 | 3 |
| Glyceryl Glucoside | 3.5 | 3 | 1 | 1 | 2 | | | 2 |
| Alcohol denat. | 1 | 3 | 0.5 | 10 | 4 | 8 | | 4 |
| Butylene Glycol | | | 3 | | | | | |
| Ascorbylglucoside | | 0.5 | | 1.0 | | 1.5 | | 0.1 |
| Ubiquinone (Coenzyme 10) | 0.1 | | 0.05 | | | | 0.01 | |
| Hyaluronic acid | | | | | 0.2 | | | |
| Bisabolol | 0.5 | | | | | | 0.2 | |
| Isotridecylsalicylate | | | 1 | 3 | 5 | 2 | 3 | 5 |
| MA-Gly or MA-PDO | 0.01 to 3.0 | | | | | | | |
| Dibutyl Adipate | 1.5 | 3 | | | | | | |
| Diisopropyl sebacate | | 1 | 1 | 2 | 3 | | | |
| Ethylhexyl Benzoate | | | | | | 0.75 | 1.5 | 1 |
| Titanium Dioxide (PARSOL TX) | | | 0.5 | 2 | | | | |
| Methylene Bis-Benztriazoyl Tetramethylbutylphenol | | | 0.5 | 4 | | 6 | | 2 |
| Ethylhexyl methoxycinnamate | | | | | 2 | | | |
| Phenylbenzimidazole Sulfonic Acid | | | | 2 | | 2 | 2 | |
| Butyl Methoxydibenzoylmethane | | 1 | | 2 | 2 | 3 | 3 | 3 |
| Methylbenzylidene Camphor | | | | | 2 | 3 | | |
| Octocrylene | | 5 | | | | 2 | 10 | |
| Polysilicone-15 | | | | 2 | | 3 | | |
| Ethylhexyl Salicylate | | | | | 5 | | | |
| Homosalate | | | 4 | | 2 | | | |
| Bis-Ethylhexyloxyphenol Methoxyphenyltriazine | | 1.5 | | | | | | 2 |
| Silica | 1 | | 2.5 | | | 0.5 | | |
| Silica & Methicone | | 4 | | 1 | 2.5 | | | |
| Methyl Methacrylate Crosspolymer | | | | 1 | | | 2 | |
| Disodium EDTA | 0.1 | | | | | 0.5 | | |
| Fragrance, Preservatives | q.s. | | | | | | | |
| Sodium Hydroxide | q.s. | | | | | | | |
| Water | Ad 100 | | | | | | | |

## Claims

1. Mandelic acid addition salt of palmitoyl lysylvalyl-lysine.

2. The mandelic acid addition salt of claim 1, wherein the palmitoyl lysylvalyl-lysine is N²-(1-oxohexadecyl)-L-lysyl-L-valyl-L-Lysine.

3. The mandelic acid addition salt of claim 1 and/ or 2, wherein the mandelic acid is D-(-)-mandelic acid, L-(+)-mandelic acid or D,L-mandelic acid, preferably D,L-mandelic acid.

4. The mandelic acid addition salt according to anyone of the preceding claims, wherein the mol equivalents of mandelic acid in the mandelic acid addition salt is selected in the range of 0.5 to 3 mol equivalents, based on palmitoyl lysylvalyl-lysine.

5. The mandelic acid addition salt according to anyone of the preceding claims, wherein the salt is prepared by dissolving palmitoyl lysylvalyl-lysine acetate in a C₁₋₆ alkyl alcohol and/ or acetic acid to form a peptide acetate solution, combining said peptide acetate solution with a solution of mandelic acid in a C₁₋₆ alkyl alcohol and/ or acetic acid, followed by removal of the C₁₋₆ alkyl alcohol and/ or acetic acid by evaporation and/ or lyophilization.

6. The mandelic acid addition salt according to claim 5, where the C₁₋₆ alkyl alcohol is methanol.

7. The mandelic acid addition salt according to anyone of the preceding claims, wherein the addition salt is comprised in a composition further comprising at least one water-soluble polyol containing 2 to 10 carbon atoms and 2 to 7 hydroxyl groups and water.

8. The mandelic acid addition salt according to claim 7, wherein the composition comprises
(i) from 0.001-12.5 wt.-%, particularly 0.01-2 wt.-%, most particularly 0.1-0.5 wt.-% of the mandelic acid addition salt of palmitoyl lysylvalyl-lysine,
(ii) up to 99.999 wt.-%, particularly 40-80 wt.-%, most particularly 60-75 wt.-% of at least one water-soluble polyol containing 2 to 10 carbon atoms and 2 to 7 hydroxyl groups and
(iv) up to 90 wt.-%, particularly up to 50 wt.-%, most particularly 0-35wt.-% of water.

9. The mandelic acid addition salt according to claim 8, wherein the ingredients (i) to (iii) of the composition sum up to 100 wt.-%.

10. The mandelic acid addition salt according to any one of claim 7 to 9, wherein the at least one water-soluble polyol containing 2 to 10 carbon atoms and 2 to 7 hydroxyl groups is selected from the group consisting of glycerin, 1,2-propylene glycol, 1,3-propanediol and/ or 1,4-butylene glycol, preferably from glycerin and 1,3-propanediol, most preferably glycerin.

11. The mandelic acid addition salt according to anyone of the preceding claims, wherein the addition salt is comprised in a blend consisting essentially of
(a) 0.05-1 wt.-%, preferably 0.1 to 1 wt.-%, most preferably 0.2 to 0.5 wt.-% of the mandelic acid addition salt,
(b) 15-50 wt.-%, preferably 20 to 40 wt.-%, most preferably 25 to 35 wt.-% of panthenol,
(c) 0.1-1 wt.-%, preferably 0.2 to 0.8 wt.-%, most preferably 0.25 to 0.7wt.-% of sodium hyaluronate,
(d) 0.25-7 wt.-%, preferably 0.3 to 5 wt.-%, most preferably 0.5 to 3 wt.-% of at least one algae extract, preferably a *Dunaliella Salina extract,*
(e) 0.25-7 wt.-%, preferably 0.3 to 5 wt.-%, most preferably 0.5 to 3 wt.-% of pentylene glycol,
(f) up to 1 wt.-%, preferably 0.001 to 1 wt.-%, most preferably 0.001 to 0.1 wt.-% of citric acid, and
(g) at least 50 wt.-% of water.

12. A cosmetic preparation comprising a mandelic acid addition salt of palmitoyl lysylvalyl-lysine according to any one of claims 1 to 11 and a cosmetically acceptable carrier.

13. The cosmetic composition according to claim 12, wherein the carrier consists of at least 50 wt.-% of water.

14. The cosmetic composition according to claim 12 to 13, wherein the composition is in the form of a gel or an emulsion.

15. Use of mandelic acid to increase the storage stability of palmitoyl lysylvalyl-lysine, preferably in a composition comprising at least one water-soluble polyol containing 2 to 10 carbon atoms and 2 to 7 hydroxyl groups and/ or a cosmetic preparation comprising a cosmetically acceptable carrier.

16. Use according to claim 15, wherein the at least one water soluble polyol is glycerin or 1,3-propanediol.

## Patentansprüche

1. Mandelsäureadditionssalz von Palmitoyllysylvalyllysin.

2. Mandelsäureadditionssalz nach Anspruch 1, wobei es sich bei dem Palmitoyllysylvalyllysin um N²-(1-Oxohexadecyl)-L-lysyl-L-valyl-L-lysin handelt.

3. Mandelsäureadditionssalz nach Anspruch 1 und/oder 2, wobei es sich bei der Mandelsäure um D-(-)-Mandelsäure, L-(+)-Mandelsäure oder D,L-Mandelsäure, vorzugsweise D,L-Mandelsäure, handelt.

4. Mandelsäureadditionssalz nach einem der vorhergehenden Ansprüche, wobei die Moläquivalente von Mandelsäure in dem Mandelsäureadditionssalz im Bereich von 0,5 bis 3 Moläquivalenten, bezogen auf Palmitoyllysylvalyllysin, ausgewählt sind.

5. Mandelsäureadditionssalz nach einem der vorhergehenden Ansprüche, wobei das Salz durch Lösen von Palmitoyllysylvalyllysinacetat in einem C₁₋₆-Alkylalkohol und/oder Essigsäure unter Bildung einer Peptidacetatlösung, Kombinieren der Peptidacetatlösung mit einer Lösung von Mandelsäure in einem C₁₋₆-Alkylalkohol und/oder einer Essigsäure, anschließendes Entfernen des C₁₋₆-Alkylalkohols und/oder der Essigsäure durch Eindampfen und/oder Lyophilisierung, hergestellt wird.

6. Mandelsäureadditionssalz nach Anspruch 5, wobei es sich bei dem C₁₋₆-Alkylalkohol um Methanol handelt.

7. Mandelsäureadditionssalz nach einem der vorhergehenden Ansprüche, wobei das Additionssalz in einer Zusammensetzung enthalten ist, die ferner mindestens ein wasserlösliches Polyol mit 2 bis 10 Kohlenstoffatomen und 2 bis 7 Hydroxylgruppen und Wasser umfasst.

8. Mandelsäureadditionssalz nach Anspruch 7, wobei die Zusammensetzung Folgendes umfasst:
(i) 0,001-12,5 Gew.-%, insbesondere 0,01-2 Gew.-%, ganz besonders 0,1-0,5 Gew.-% Mandelsäureadditionssalz von Palmitoyllysylvalyllysin,
(ii) bis zu 99,999 Gew.-%, insbesondere 40-80 Gew.-%, ganz besonders 60-75 Gew.-% mindestens eines wasserlöslichen Polyol mit 2 bis 10 Kohlenstoffatomen und 2 bis 7 Hydroxylgruppen und
(iv) bis zu 90 Gew.-%, insbesondere bis zu 50 Gew.-%, ganz besonders 0-35 Gew.-% Wasser.

9. Mandelsäureadditionssalz nach Anspruch 8, wobei sich die Inhaltsstoffe (i) bis (iii) der Zusammensetzung zu 100 Gew.-% summieren.

10. Mandelsäureadditionssalz nach einem der Ansprüche 7 bis 9, wobei das mindestens eine wasserlösliche Polyol mit 2 bis 10 Kohlenstoffatome und 2 bis 7 Hydroxylgruppen aus der Gruppe bestehend aus Glycerin, 1,2-Propylenglykol, 1,3-Propandiol und/oder 1,4-Butylenglykol, vorzugsweise aus Glycerin und 1,3-Propandiol, am meisten bevorzugt Glycerin ausgewählt ist.

11. Mandelsäureadditionssalz nach einem der vorhergehenden Ansprüche, wobei das Additionssalz in einer Mischung enthalten ist, die im Wesentlichen aus Folgendem besteht:
(a) 0,05-1 Gew.-%, vorzugsweise 0,1 bis 1 Gew.-%, am meisten bevorzugt 0,2 bis 0,5 Gew.-% Mandelsäureadditionssalz,
(b) 15-50 Gew.-%, vorzugsweise 20 bis 40 Gew.-%, am meisten bevorzugt 25 bis 35 Gew.-% Panthenol,
(c) 0,1-1 Gew.-%, vorzugsweise 0,2 bis 0,8 Gew.-%, am meisten bevorzugt 0,25 bis 0,7 Gew.-% Natriumhyaluronat,
(d) 0,25-7 Gew.-%, vorzugsweise 0,3 bis 5 Gew.-%, am meisten bevorzugt 0,5 bis 3 Gew.-% mindestens eines Algenextrakts, vorzugsweise eines Dunaliella-Salina-Extrakts,
(e) 0,25-7 Gew.-%, vorzugsweise 0,3 bis 5 Gew.-%, am meisten bevorzugt 0,5 bis 3 Gew.-% Pentylenglykol,
(f) bis zu 1 Gew.-%, vorzugsweise 0,001 bis 1 Gew.-%, am meisten bevorzugt 0,001 bis 0,1 Gew.-% Citronensäure und
(g) mindestens 50 Gew.-% Wasser.

12. Kosmetische Zubereitung, umfassend ein Mandelsäureadditionssalz von Palmitoyllysylvalyllysin nach einem der Ansprüche 1 bis 11 und einen kosmetisch unbedenklichen Träger.

13. Kosmetische Zusammensetzung nach Anspruch 12, wobei der Träger aus mindestens 50 Gew.-% Wasser besteht.

14. Kosmetische Zusammensetzung nach Anspruch 12 oder 13, wobei die Zusammensetzung in Form eines Gels oder einer Emulsion vorliegt.

15. Verwendung von Mandelsäure zum Erhöhen der Lagerstabilität von Palmitoyllysylvalyllysin, vorzugsweise in einer Zusammensetzung, die mindestens ein wasserlösliches Polyol mit 2 bis 10 Kohlenstoffatomen und 2 bis 7 Hydroxylgruppen umfasst, und/oder einer kosmetischen Zubereitung, die einen kosmetisch unbedenklichen Träger umfasst.

16. Verwendung nach Anspruch 15, wobei es sich bei dem mindestens einen wasserlöslichen Polyol um Glycerin oder 1,3-Propandiol handelt.

## Revendications

1. Sel d'addition d'acide mandélique de palmitoyl-lysylvalyl-lysine.

2. Sel d'addition d'acide mandélique selon la revendication 1, dans lequel la palmitoyl-lysylvalyl-lysine est la N²-(1-oxohexadécyl)-L-lysyl-L-valyl-L-lysine.

3. Sel d'addition d'acide mandélique selon la revendication 1 et/ou 2, dans lequel l'acide mandélique est l'acide D-(-)-mandélique, l'acide L-(+)-mandélique ou l'acide D,L-mandélique, de préférence l'acide D,L-mandélique.

4. Sel d'addition d'acide mandélique selon l'une quelconque des revendications précédentes, dans lequel les équivalents molaires d'acide mandélique dans le sel d'addition d'acide mandélique sont choisis dans la plage de 0,5 à 3 équivalents molaires, par rapport à la palmitoyl-lysylvalyl-lysine.

5. Sel d'addition d'acide mandélique selon l'une quelconque des revendications précédentes, dans lequel le sel est préparé en dissolvant de l'acétate de palmitoyl-lysylvalyl-lysine dans un alcool alkylique en C₁₋₆ et/ou de l'acide acétique pour former une solution d'acétate de peptide, en combinant ladite solution d'acétate de peptide avec une solution d'acide mandélique dans un alcool alkylique en C₁₋₆ et/ou de l'acide acétique, suivi par élimination de l'alcool alkylique en C₁₋₆ et/ou de l'acide acétique par évaporation et/ou lyophilisation.

6. Sel d'addition d'acide mandélique selon la revendication 5, dans lequel l'alcool alkylique en C₁₋₆ est le méthanol.

7. Sel d'addition d'acide mandélique selon l'une quelconque des revendications précédentes, dans lequel le sel d'addition est compris dans une composition comprenant en outre au moins un polyol hydrosoluble contenant 2 à 10 atomes de carbone et 2 à 7 groupes hydroxyle et de l'eau.

8. Sel d'addition d'acide mandélique selon la revendication 7, dans lequel la composition comprend
(i) de 0,001 à 12,5 % en poids, en particulier de 0,01 à 2 % en poids, le plus particulièrement de 0,1 à 0,5 % en poids du sel d'addition d'acide mandélique de palmitoyl-lysylvalyl-lysine,
(ii) jusqu'à 99,999 % en poids, en particulier 40 à 80 % en poids, le plus particulièrement 60 à 75 % en poids d'au moins un polyol hydrosoluble contenant 2 à 10 atomes de carbone et 2 à 7 groupes hydroxyle et
(iii) jusqu'à 90 % en poids, en particulier jusqu'à 50 % en poids, le plus particulièrement 0 à 35 % en poids d'eau.

9. Sel d'addition d'acide mandélique selon la revendication 8, dans lequel les ingrédients (i) à (iii) de la composition totalisent jusqu'à 100 % en poids.

10. Sel d'addition d'acide mandélique selon l'une quelconque des revendications 7 à 9, dans lequel l'au moins un polyol hydrosoluble contenant 2 à 10 atomes de carbone et 2 à 7 groupes hydroxyle est choisi dans le groupe constitué par la glycérine, le 1,2-propylène glycol, le 1,3-propanediol et/ou le 1,4-butylène glycol, de préférence parmi la glycérine et le 1,3-propanediol, et le plus préférablement la glycérine.

11. Sel d'addition d'acide mandélique selon l'une quelconque des revendications précédentes, dans lequel le sel d'addition est compris dans un mélange essentiellement constitué de
(a) 0,05 à 1 % en poids, de préférence 0,1 à 1 % en poids, le plus préférablement 0,2 à 0,5 % en poids de sel d'addition d'acide mandélique,
(b) 15 à 50 % en poids, de préférence 20 à 40 % en poids, le plus préférablement 25 à 35 % en poids de panthénol,
(c) 0,1 à 1 % en poids, de préférence 0,2 à 0,8 % en poids, le plus préférablement 0,25 à 0,7 % en poids de hyaluronate de sodium,
(d) 0,25 à 7 % en poids, de préférence 0,3 à 5 % en poids, le plus préférablement 0,5 à 3 % en poids d'au moins un extrait d'algues, de préférence un extrait de *Dunaliella Salina*,
(e) 0,25 à 7 % en poids, de préférence 0,3 à 5 % en poids, le plus préférablement 0,5 à 3 % en poids de pentylène glycol,
(f) jusqu'à 1 % en poids, de préférence 0,001 à 1 % en poids, le plus préférablement 0,001 à 0,1 % en poids d'acide citrique, et
(g) au moins 50 % en poids d'eau.

12. Préparation cosmétique comprenant un sel d'addition d'acide mandélique de palmitoyl-lysylvalyl-lysine selon l'une quelconque des revendications 1 à 11 et un support cosmétiquement acceptable.

13. Composition cosmétique selon la revendication 12, dans laquelle le support est constitué d'au moins 50 % en poids d'eau.

14. Composition cosmétique selon les revendications 12 à 13, dans laquelle la composition est sous la forme d'un gel ou d'une émulsion.

15. Utilisation d'acide mandélique pour augmenter la stabilité au stockage de palmitoyl-lysylvalyl-lysine, de préférence dans une composition comprenant au moins un polyol hydrosoluble contenant 2 à 10 atomes de carbone et 2 à 7 groupes hydroxyle et/ou une préparation cosmétique comprenant un support cosmétiquement acceptable.

16. Utilisation selon la revendication 15, dans laquelle l'au moins un polyol soluble dans l'eau est la glycérine ou le 1,3-propanediol.
